# EUROPEAN PATENT APPLICATION

(11) **EP 2 181 681 A1**
(43) Date of publication of application: **05.05.2010**
(21) Application number: 08791236.6
(22) Date of filing: 16.07.2008
(51) Int. Cl.: A61F 13/15, A61F 13/49, A61F 13/511

(54) **DISPOSABLE DIAPER**

(30) Priority: 03.08.2007 JP 2007203376
(71) Applicant: Unicharm Corporation, Ehime 799-0111 (JP)
(72) Inventor: SAKAGUSHI, Satoru c/o Technical Center of UNI-CHA, Kanonji-shi, Kagawa 769-1602 (JP); YOSHIMASA, Wataru c/o Technical Center of UNI-CHA, x (JP); HISANAKA, Takayuki c/o Technical Center of UNI-CHA, Kanonji-shi, Kagawa 769-1602 (JP)
(74) Representative: Peter, Julian
(86) International application number: PCT/JP2008/062841
(87) International publication number: WO 2009/019962

(57) **Abstract**

A liquid-permeating sheet (17) having an antibacterial-containing polymer coating part (31) disposed on the side opposite to the skin side is bonded to a back waist area of a diaper base. When a wearer sweats, the sweat penetrates the antibacterial-containing polymer part (31) through the liquid-permeating sheet (17). The antibacterial contained in the antibacterial-containing polymer containing part (31) then comes into the sweat to inhibit the propagation of *Staphylococcus epidermidis.*

## Description

### TECHNICAL FIELD

The present invention relates to a disposable diaper which effectively prevents a rash such as a heat rash.

### BACKGROUND ART

Heretofore, it has been known that a wearer wearing a disposable diaper is likely to get a rash, such as a heat rash, around a waist or the like of the wearer. Especially in the summer time when sweat is increased, the wearer is likely to get a heat rash on the back of the wearer with which a back waistline region of the diaper comes into contact. One of possible causes of the heat rash occurrence having been reported so far is that the sweat duct of the wearer is blocked by the sweat, the dust, or the like on a skin of the wearer.

A disposable diaper made to prevent the heat rash has been conventionally known which has a sweat-absorptive, air-permeable, liquid-permeable sheet bonded to a skin facing side or the like of the back waistline region thereof (see Patent Documents 1 and 2, for example).
Patent Document 1: Japanese Patent Application Publication No. 2000-189454
Patent Document 2: Japanese Patent Application Publication No. 2004-358099

### DISCLOSURE OF THE INVENTION

However, the recent study has revealed that the excessive growth of *Staphylococcus epidermidis,* which inherently exists on the skin, due to the large amount of sweating is one of the causes of the heat rash occurrence.

Staphylococcus epidermidis is an indigenous bacterium, and is a so-called "friendly" bacterium which inhibits the growth of Staphylococcus aureus or the like, Staphylococcus aureus causing a skin disease. Under the normal condition, Staphylococcus epidermidis does not grow excessively, thus does not cause the heat rash. However, Staphylococcus epidermidis grows excessively when there is a large amount of sweating remaining on the skin as in a diaper in the summer time, and thus causes the heat rash on the skin of the wearer.

However, it is difficult to prevent the excessive growth of *Staphylococcus epidermidis* by using the techniques described in the above-mentioned Patent Documents 1 and 2, although these techniques allow the absorption of the sweat. Therefore, the techniques have a problem of failing to prevent the heat rash sufficiently.

Accordingly, an object of the present invention is to provide a disposable diaper to effectively inhibit the occurrence of a rash, such as a heat rash.

According to claim 1 of the present invention, a disposable diaper comprising a diaper main body which includes an inner surface sheet, an outer surface sheet, and a liquid absorbent core, and which has a waistline region and a leg-surrounding region formed therein, the inner surface sheet disposed on a skin facing side of the disposable diaper, the skin facing side facing a skin of a wearer when the disposable diaper is worn, the outer surface sheet opposed to the inner surface sheet and disposed on a non-skin facing side which is the opposite side to the skin facing side, the liquid absorbent core interposed between the inner surface sheet and the outer surface sheet, wherein a liquid-permeable portion which allows moisture to permeate therethrough is provided at least partially on a skin facing side of any of the waistline region and the leg-surrounding region of the diaper main body, the skin facing side facing the skin of the wearer, and an antibacterial-agent-containing polymer coated portion coated with an antibacterial-agent-containing polymer is provided between the outer surface sheet and a skin-facing surface of the liquid-permeable portion, the antibacterial-agent-containing polymer containing a thermoplastic water-soluble polymer and an antibacterial agent, the skin-facing surface being a surface of the liquid-permeable portion on the skin facing side.

In the invention according to claim 1, an antibacterial-agent-containing polymer coated portion coated with an antibacterial-agent-containing polymer is provided between an outer surface sheet and a skin-facing surface of the liquid-permeable portion, the skin-facing surface being a surface of the liquid-permeable portion on the skin facing side. Thus, when a wearer sweats, the sweat permeates the liquid-permeable portion and reaches the antibacterial-agent-containing polymer coated portion. Here, the antibacterial-agent-containing polymer coated portion contains a water-soluble polymer and an antibacterial agent. Accordingly, the water-soluble polymer in the antibacterial-agent-containing polymer coated portion is dissolved and thus the antibacterial agent is released into the liquid-permeable portion. The released antibacterial agent inhibits the excessive growth of *Staphylococcus epidermidis* on the skin of the wearer, and thus allows an effective prevention of the rash, such as a heat rash.

It should be noted that the skin of the wearer comes into direct contact with the skin-facing surface of the liquid-permeable portion. Thus, if the antibacterial-agent-containing polymer portion is coated on the skin-facing surface, the antibacterial agent is released even with the small amount of sweating. Here, an appropriate extent of *Staphylococcus epidermidis* has to exist on the skin, since *Staphylococcus epidermidis* is an effective, "friendly" bacterium, as described above. For this reason, it is not preferable that the small amount of sweating causes the release of the antibacterial agent and that the released antibacterial agent exert its antibacterial effect on *Staphylococcus epidermidis.* Accordingly, as in the present invention, it is preferable to provide the antibacterial-agent-containing polymer coated portion between the outer surface sheet and a skin-facing surface of the liquid-permeable portion.

### BRIEF DESCRIPTION OF THE DRAWINGS

[FIG. 1] FIG. 1 is a perspective view showing a disposable diaper according to an embodiment of the present invention.
[FIG. 2] FIG. 2 is a plan view showing the spread disposable diaper in FIG. 1.
[FIG. 3] FIG. 3 is a cross-sectional view taken along the line A-A in FIG. 2.
[FIG. 4] FIG. 4 is a cross-sectional view equivalent to FIG. 3, according to a different embodiment.
[FIG. 5] FIG. 5 is a cross-sectional view of a liquid-permeable sheet formed of two layers.
[FIG. 6] FIG. 6 is a cross-sectional view of a liquid-permeable sheet formed of three layers.
[FIG. 7] FIG. 7 is a plan view of an open-type diaper on which a liquid-permeable sheet is pasted.
[FIG. 8] FIG. 8 is a plan view of an open-type diaper on which a liquid-permeable sheet is pasted.
[FIG. 9] FIG. 9 is a plan view of an open-type diaper on which a liquid-permeable sheet is pasted.
[FIG. 10] FIG. 10 is a plan view of an open-type diaper on which a liquid-permeable sheet is pasted with a hydrophobic sheet interposed in between.
[FIG. 11] FIG. 11 is a plan view of an open-type diaper on which a hydrophobic sheet is pasted.
[FIG. 12] FIG. 12 is a plan view of an open-type diaper in which an antibacterial-agent-containing polymer coated portion is provided on a non-skin facing side of an inner surface sheet.
[FIG. 13] FIG. 13 is a cross-sectional view illustrating an antibacterial effect of the liquid-permeable sheet. Part (a) of FIG. 13 shows the state before sweat absorption, Part (b) of FIG. 13 shows the state where the liquid-permeable sheet gets wet with sweat, and Part (c) of FIG. 13 shows the state where the antibacterial agent inside the antibacterial-agent-containing polymer coated portion is dispersed by the sweat.
[FIG. 14] FIG. 14 is a schematic diagram showing a state of the skin of a wearer, in Example Case.
[FIG. 15] FIG. 15 is a schematic diagram showing a temperature distribution of the skin of a wearer, in Example Case.

### BEST MODE FOR CARRYING OUT THE INVENTION

An embodiment of the present invention will be described below.

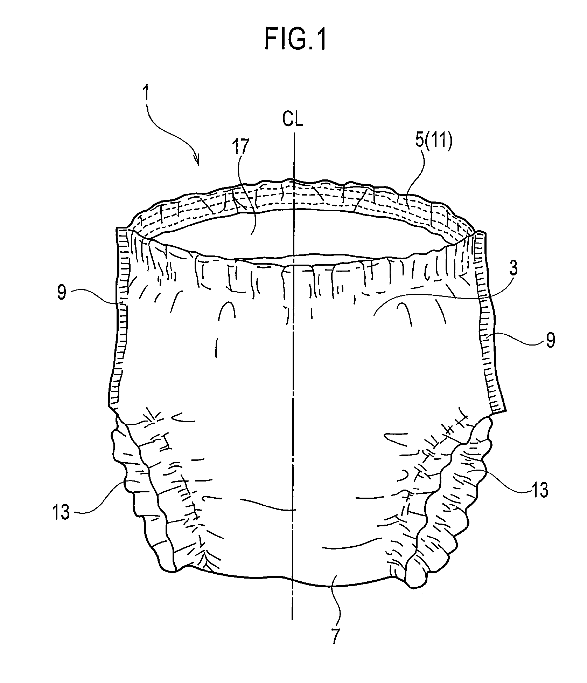

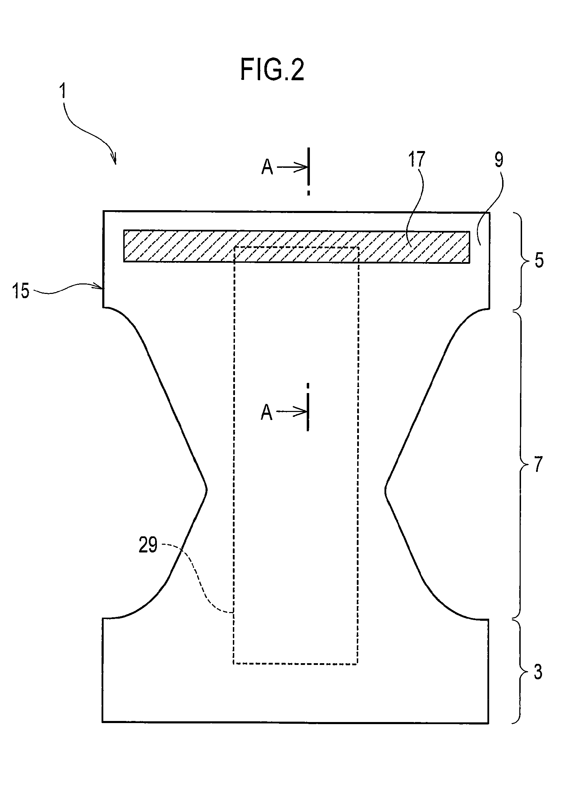

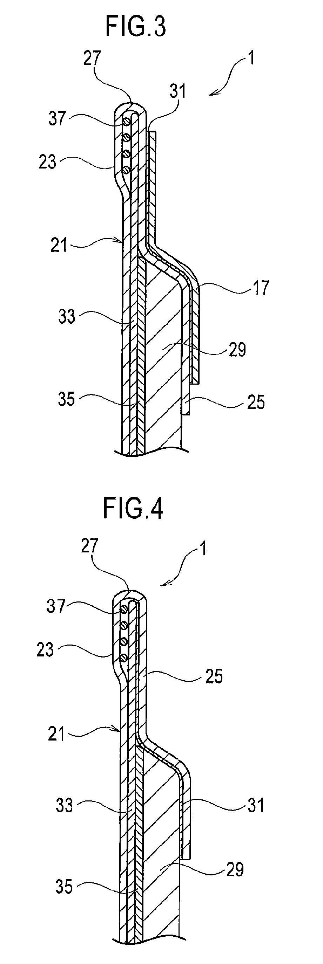

FIG. 1 is a perspective view showing a disposable diaper according to an embodiment of the present invention. FIG. 2 is a plan view showing the disposable diaper in FIG. 1 spread by tearing right-and-left-side edge portions, the disposable diaper seen from a skin facing side which comes into contact with the skin of a wearer when the diaper is worn. Meanwhile, FIG. 3 is a cross-sectional view taken along the line A-A in FIG. 2.

As shown in FIG. 1, the disposable diaper is a pants-type diaper 1 which is formed symmetrically with respect to an axial center line CL. Note that the present invention is also applicable to an open-type diaper, in which right-and-left side edge portions are not joined, to be described later.

The pants-type diaper 1 includes a diaper main body 15 (see FIG. 2) which is formed of a waistline region, a crotch region 7, and a leg-surrounding region 13. The waistline region includes a front waistline region 3 and a back waistline region 5. Right-and-left side edge portions 9, 9 of the front waistline region 3 are joined to right-and-left side edge portions 9, 9 of the back waistline region 5, respectively. A waistline-side opening portion 11 is formed on the top end of the diaper main body 1.
As shown in FIG. 2, the pants-type diaper 1 according to the present embodiment includes the diaper main body 15 and a liquid-permeable sheet (liquid-permeable portion) 17. The diaper main body 15 absorbs and holds excrement and body fluids such as sweat. The liquid-permeable sheet 17 is attached to a skin facing side of the back waistline region 5 of the diaper main body 15. The liquid-permeable sheet 17 is set as a liquid-permeable portion which allows moisture to permeate therethrough. The liquid-permeable sheet 17 may be provided, not only on the skin facing side of the back waistline region 5, but also on any of a skin facing side of the front waistline region 3 and a skin facing side of the leg-surrounding region 13. More specifically, the skin facing side is referred to as a side facing the skin of the wearer when a wearer wears the diaper, and the non-skin facing side is referred to as a side opposite to the skin facing side.

Here, the liquid-permeable portion indicates a region in which the water resistance is 100 mm or less provided that a surface tension regulator is added to distilled water so that the mixture has the surface tension adjusted to 30 mN/m. The liquid-permeable portion also includes the one formed of a hydrophobic material. To be more specific, for the liquid-permeable portion, it is preferable to use a nonwoven fabric containing hydrophilic fibers, a sheet obtained by hydrophilizing a nonwoven fabric containing hydrophilic fibers, or a sheet obtained by hydrophilizing a nonwoven fabric containing hydrophobic fibers. It is more preferable to use a tissue paper or a spun-lace nonwoven fabric. The tissue paper or the spun-lace nonwoven fabric secures its sheet strength by hydrogen bonding. If the tissue paper or the like is soaked in a solution containing an antibacterial agent here, the hydrogen bonding of the tissue paper or the like is deteriorated. This decreases the strength of the tissue paper or the like, thereby likely leading to a problem of a fluffy surface or the like. Therefore, it is preferable that the liquid-permeable portion be formed of the tissue paper or the spun-lace nonwoven fabric and then the liquid-permeable portion be coated with an antibacterial-agent-containing polymer.

As shown in FIG. 3, an inner surface sheet 25 is provided on the skin facing side, while an outer surface sheet 23 is provided on the non-skin facing side. In FIG. 3, the outer surface sheet 23 and the inner surface sheet 25 are formed of a single sheet. In FIG. 3, the outer surface sheet 23 extends upward and reaches a top end portion 27. Meanwhile, the inner surface sheet 25 is a folded-over portion obtained by folding over the top end portion 27. The inner surface sheet 25 extends downward from the top end portion and thus covers an upper side of a liquid absorbent core 29. The liquid-permeable sheet 17, being the liquid-permeable portion, is attached to a skin facing side of the inner surface sheet 25. An antibacterial-agent-containing polymer coated portion 31 is formed on a non-skin facing side of the liquid-permeable sheet 17. The antibacterial-agent-containing polymer coated portion 31 is pasted to the skin facing side of the inner surface sheet 25 by use of an adhesive which is not shown in the figure. An inner sheet 33 is provided on a skin facing side of the outer surface sheet 23. Between the inner sheet 33 and the liquid absorbent core 29, a film 35 is provided to prevent moisture of the excrement or the like from leaking to the outside. Moreover, between an upper end portion of the outer surface sheet 23 and an upper end portion of the inner sheet 33, an elastic body 37 made of rubber or the like is provided. Here, the antibacterial-agent-containing polymer coated portion 31 may be provided on the skin facing side of a portion of the inner surface sheet 25, the portion facing the liquid-permeable sheet 17.

Since the liquid-permeable sheet 17 is configured separately from the inner surface sheet 25 in the above-mentioned manner, it is possible to easily change the height, the width, or the thickness of the liquid-permeable sheet 17. For example, a liquid-permeable sheet 17 having a large thickness may be used if an antibacterial agent is desired to be dispersed by causing a water-soluble polymer to be dissolved with a large amount of sweat. It is preferable that the liquid-permeable sheet contain cellulose fibers and that the antibacterial agent be a cationic surfactant. Moreover, it is preferable that a tissue paper or a spun-lace nonwoven fabric be employed for the liquid-permeable sheet. Here, even a nonwoven fabric made of hydrophobic fibers or a sheet obtained by hydrophilizing a nonwoven fabric containing hydrophobic fibers may be used for the liquid-permeable sheet, since such the fabric or sheet still can cause the sweat to permeate therethrough and absorb the sweat.

Note that, for the clarification of the antibacterial-agent-containing polymer coated portion 31, the description has been given as if the antibacterial-agent-containing polymer coated portion 31 is formed as a layer. However, the antibacterial-agent-containing polymer coated portion 31 is a portion in which an antibacterial-agent-containing polymer in the form of particulates attaches to fibers constituting the liquid-permeable sheet 17. Also in the drawings to be described, the antibacterial-agent-containing polymer coated portion 31 is to be shown as a layer for the simplicity. Here, coating the antibacterial-agent-containing polymer in a dotted pattern to the liquid-permeable portion as described above has an advantage that the water-soluble polymer can be effectively dissolved by the absorbed sweat.

In addition, a structure shown in FIG. 4 may be employed in the present invention.

In this structure, the inner surface sheet 25 being the folded-over portion is set as the liquid-permeable portion, and the antibacterial-agent-containing polymer coated portion 31 is provided on the non-skin facing side of the inner surface sheet 25. A nonwoven fabric made of hydrophobic fibers may be used for the inner surface sheet 25. Even such the sheet gets wet by the sweat and can cause the moisture to permeate therethrough. Here, the inner surface sheet 25 which is hydrophilized or perforated is further preferable because such the inner surface sheet 25 causes the sweat or the like to permeate therethrough more easily. Accordingly, by forming the antibacterial-agent-containing polymer coated portion 31 on the non-skin facing side of the inner surface sheet 25, the sweat absorbed into the inner surface sheet 25 permeates the antibacterial-agent-containing polymer coated portion 31. Thus, the antibacterial agent in the antibacterial-agent-containing polymer coated portion 31 can be released.

Note that the antibacterial-agent-containing polymer coated portion 31 may be provided on the skin facing side of a portion of the diaper main body 15 (the inner sheet 33 and the absorbent core 29), the portion facing the inner surface sheet 25. Providing the inner surface sheet 25, being the folded-over portion, in this manner in the liquid-permeable portion and providing the antibacterial-agent-containing polymer coated portion 31 on the non-skin facing side of the inner surface sheet 25 has an advantage that a simple structure with fewer components can be obtained.

Although not shown, the liquid-permeable sheet 17 may be disposed between the non-skin facing side of the inner surface sheet 25 and the skin facing side of the diaper main body 15. In other words, such a structure may be employed in which the liquid-permeable sheet 17 is pasted to a range across the skin facing side of the upper portion of the liquid absorbent core 29 shown in FIG. 4 and the skin facing side of the top end portion of the inner sheet 33 shown in FIG. 4, and in which the inner surface sheet 25 covers the skin facing side of the liquid-permeable sheet 17 at least partially. Here, the liquid-permeable sheet 17 and a portion of the inner surface sheet 25 are set as the liquid-permeable portion, the portion covering the liquid-permeable sheet 17.

In this case, as to the antibacterial-agent-containing polymer coated portion 31, the antibacterial-agent-containing polymer coated portion is provided on at least any one of the non-skin facing side of the portion of the inner surface sheet 25 covering the liquid-permeable sheet 17, the skin facing side of the liquid-permeable sheet 17, the non-skin facing side of the liquid-permeable sheet 17, and the skin facing side of the portion of the diaper main body (the upper portion of the liquid absorbent core 29 and the top end portion of the inner sheet 33) facing the liquid-permeable sheet 17. The structure in which the inner surface sheet 25 covers the skin facing side of the liquid-permeable sheet 17 at least partially in this manner can prevent displacement of the liquid-permeable sheet 17, because the liquid-permeable sheet 17 does not come into direct contact with the skin of the wearer.

Furthermore, the inner surface sheet 25 shown in FIG. 4 is formed of the folded-over portion, but not limited to this. An inner surface sheet and an outer surface sheet may be formed separately, the inner surface sheet may be set as a liquid-permeable portion, and the antibacterial-agent-containing polymer coated portion may be provided on at least either one of a non-skin facing side of the inner surface sheet and a skin facing side of a portion of the diaper main body, the portion facing the inner surface sheet. In this structure, the inner surface sheet of the diaper main body serves as the liquid-permeable portion. Thus, a simple structure with fewer components can be obtained.

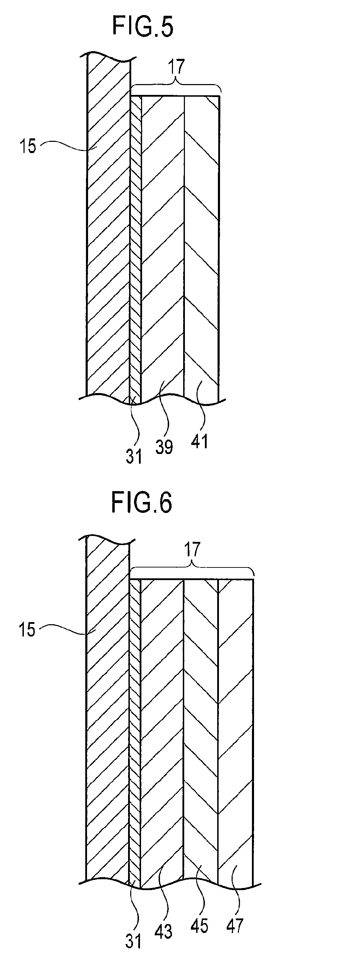

Next, a cross-sectional structure of the liquid-permeable portion will be described with reference to FIG. 5 and FIG. 6.

FIG. 5 is a cross section of a liquid-permeable sheet formed of two layers.

This liquid-permeable sheet 17 includes a skin facing side layer 41 and a cellulose layer 39, the skin facing side layer 41 disposed on the skin facing side, the cellulose layer 39 disposed on the non-skin facing side of the skin facing side layer 41 and serving as a non-skin facing side layer mainly made of cellulose fibers. The skin facing side layer 41 has lower hydrophilicity than the cellulose layer 39 does.

In addition, the antibacterial-agent-containing polymer coated portion 31 is formed on the non-skin facing side of the cellulose layer 39. In this structure, the sweat is more likely to shift from the skin facing side layer 41 to the cellulose layer 39 and thus the sweat is highly absorbed therein. This is because the structure has such a hydrophilicity gradient that the cellulose layer 39 has the higher hydrophilicity than the skin facing side layer 41 does. Note that FIG. 5 shows a case where the antibacterial-agent-containing polymer coated portion 31 is coated on the non-skin facing side of the cellulose layer 39. However, the present invention is not limited to this, and the antibacterial-agent-containing polymer coated portion 31 may be coated in such a manner as to be sandwiched between the cellulose layer 39 and the skin facing side layer 41.

FIG. 6 is a cross section of a liquid-permeable sheet formed of three layers.

This liquid-permeable sheet 17 includes a skin facing side layer 47, a cellulose layer (medium layer) 45, and a non-skin facing side layer 43, the skin facing side layer 47 disposed on the skin facing side, the cellulose layer 45 disposed on a non-skin facing side of the skin facing side layer 47 and mainly made of cellulose fibers, the non-skin facing side layer 43 disposed on a non-skin facing side of the cellulose layer 45. The hydrophilicity of the cellulose layer 45 is set higher than those of the skin facing side layer 47 and the non-skin facing side layer 43. The antibacterial-agent-containing polymer coated portion 31 is formed on a non-skin facing side of the non-skin facing side layer 43. Since the non-skin facing side layer 43 and the skin facing side layer 47 have the lower hydrophilicity than the cellulose layer 45 does, the non-skin facing side layer 43 is less likely to get wet even when the liquid-permeable sheet 17 gets wet by the sweat. Therefore, it is possible to maintain the bonding strength between the liquid-permeable sheet 17 and the diaper main body 15 at a high level.

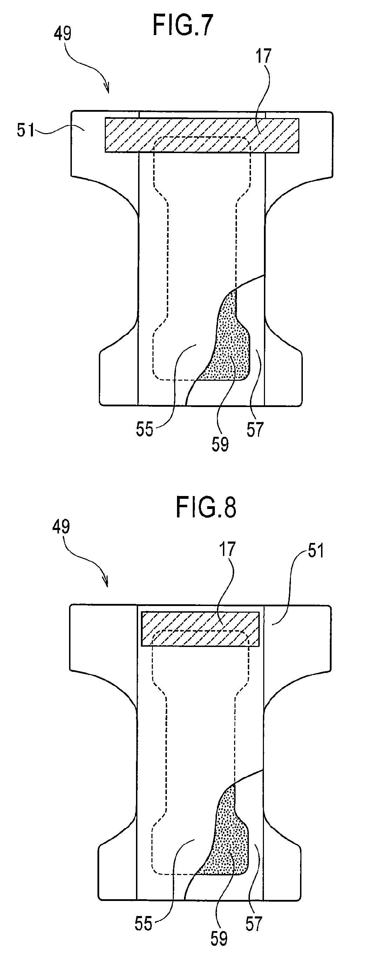

Next, by use of FIG. 7 to FIG. 9, a description will be given of a portion to which the liquid-permeable sheet 17 of the present embodiment is pasted. A disposable diaper shown here is an open-type diaper (flat type diaper).

As shown in FIG. 7 to FIG. 9, an open-type diaper 49 includes an inner surface sheet 55, an outer surface sheet 57, and a liquid absorbent core 59, the inner surface sheet 55 disposed on the skin facing side of the diaper main body, the outer surface sheet 57 disposed on the non-skin facing side, the liquid absorbent core 59 disposed between the inner surface sheet 55 and the outer surface sheet 57.

It is preferable that the liquid-permeable sheet 17 be pasted to a skin facing side of a back waistline region 51 of the open-type diaper 49 in a waistline direction as shown in FIG. 7. Specifically, it is preferable that the liquid-permeable sheet 17 be joined to a skin facing side of the inner surface sheet 55 by use of an adhesive. It is preferable that the liquid-permeable sheet 17 be in a form of a thin rectangular extending in the waistline direction and be disposed to cover 50% or more of the total length of the back waistline region 51.

Alternatively, the liquid-permeable sheet 17 may be disposed in a center portion of the back waistline region 51, as shown in FIG. 8. The length of the liquid-permeable sheet 17 in the waistline direction may be set to on the order of nearly 50% of the total length of the back waistline region.

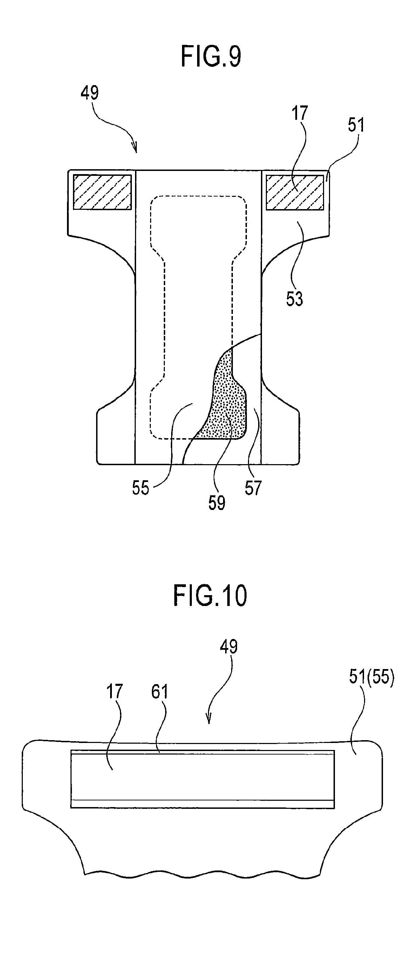

Still alternatively, the liquid-permeable sheet 17 may be pasted to a pair of right and left fixed edge portions in the open-type diaper 49, as shown in FIG. 9.

FIG. 10 to FIG. 12 show plan views showing forms of the liquid-permeable portion in the open-type diaper.

Firstly, as shown in FIG. 10, a hydrophobic sheet 61 may be pasted to the skin facing side of the back waistline region 51 of the inner surface sheet 55 which is provided on the skin facing side of the open-type diaper 49, and the liquid-permeable sheet 17 may be pasted to a skin facing side of the hydrophobic sheet 61, the skin facing side being further closer to the skin. Additionally, the antibacterial-agent-containing polymer coated portion (not shown) may be formed on at least any one of the non-skin facing side of the liquid-permeable sheet 17 and the skin facing side of the hydrophobic sheet 61. Instead, the antibacterial-agent-containing polymer coated portion may be formed on at least any one of the non-skin facing side of the hydrophobic sheet 61 and the skin facing side of the diaper main body.

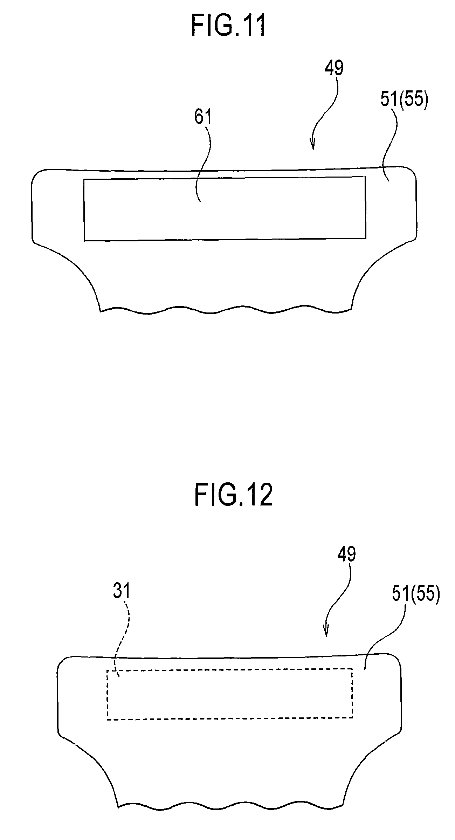

Secondly, the hydrophobic sheet 61 may be pasted to the skin facing side of the back waistline region 51 as shown in FIG. 11. In this case, the antibacterial-agent-containing polymer coated portion (not shown) is formed on at least any one of the non-skin facing side of the hydrophobic sheet 61 and the skin facing side of a portion of the diaper main body, the portion facing the hydrophobic sheet 61.

Then, as shown in FIG. 12, the antibacterial-agent-containing polymer coated portion 31 (surrounded by a dashed line) may be formed on the non-skin facing side of the inner surface sheet 55 provided on the skin facing side of the open-type diaper 49.

As has been described so far, it is preferable that the liquid-permeable portion and the antibacterial-agent-containing polymer coated portion be disposed in the back waistline region in the diaper main body because the skin facing side of the back waistline region is a part facing the back of a wearer, the back being most likely to suffer from a rash such as a heat rash. Accordingly, this disposition makes it possible to prevent the rash occurrence effectively.

Subsequently, an antibacterial action by the liquid-permeable portion will be described by taking the liquid-permeable sheet as an example.

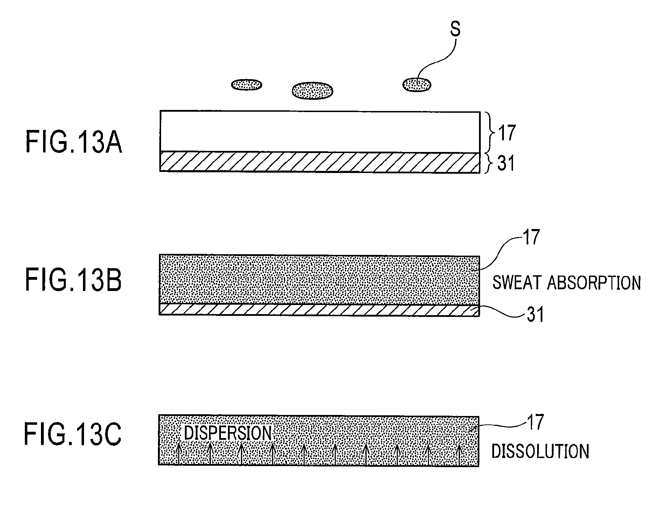

FIG. 13 is a cross section showing the antibacterial action by the liquid-permeable sheet.

As shown in Part (a) of FIG. 13, the antibacterial-agent-containing polymer coated portion 31 is formed on the non-skin facing side of the liquid-permeable sheet 17. When a sweat S of the wearer is absorbed into the liquid-permeable sheet 17, the liquid-permeable sheet 17 gets wet as shown in Part (b) of FIG. 13. Then, the water-soluble polymer in the antibacterial-agent-containing polymer coated portion 31 is dissolved by the sweat S as shown in Part (c) of FIG. 13. Accordingly, the antibacterial agent as well as the water-soluble polymer is dispersed into the liquid-permeable sheet 17 as shown by an arrow in Part (c) of FIG. 13. Thereby, *Staphylococcus epidermidis* in the sweat is treated with the antibacterial agent.

Here, it is preferable that the water-soluble polymer be formed of one or more of materials selected from the group consisting of polyethylene glycol, polyvinyl alcohol, polypropylene glycol, and derivatives of these. Also, a water-soluble polymer 1 g of which is completely dissolved in 100 cc of a soluble at 37°C, which is around the human body temperature, is preferable as the water-soluble polymer. For example, the preferable water-soluble polymer is polyethylene glycol, polyvinyl alcohol, polypropylene glycol, mixture of these, or the like. The water-soluble polymer such as polyethylene glycol is useful as a carrier to carry the antibacterial agent in the sweat.

In addition, since serving as an additive to mix the antibacterial agent therein, the water-soluble polymer is also useful as a binder to cause the antibacterial agent to attach to the liquid-permeable sheet made of a nonwoven fabric, for example. A molecular mass of the water-soluble polymer is preferably 100 to 50,000, and more preferably, 600 to 30,000.

As the antibacterial agent, any antibacterial agent can be used, as long as the antibacterial agent can suppress the growth of *Staphylococcus epidermidis,* at least. The antibacterial agent can be used in combination of two or more. Herein, the "antibacterial" represents a concept which includes partial sterilization, complete sterilization, and bacteriostasis. Note that it is more preferable that the antibacterial agent be capable of suppressing the growth of bacteria such as *Escherichia coli, Staphylococcus aureus,* and ammonia producing bacteria besides *Staphylococcus epidermidis* described above.

The followings are specific examples:

formaldehyde-releasing agents such as N,N',N"-tris(hydroxyethyl)hexahydro-s-triazine, 2-bromo-2-nitro-1,3-propanediol, 4,4-dimethyl oxazolidine, 1,3-di(hydroxymethyl)-5,5-dimethylhydantoin, tris(hydroxymethyl)nitromethane, 4-(2-nitrobutyl)morpholine, and 1,3-dimorpholino-2-nitro-2-ethylpropane;
halogen-containing compounds such as 1,3-dibromo-2,4-dicyanobutane, diiodomethyl-p-tolylsulfone, 2,3,5,6-tetrachloro-4-(methylsulfonyl)pyridine, and 2,4,5,6-tetrachloro isophthalonitrile;
iodopropagyl derivatives such as 4-chlorophenyl-3-iodopropagylformal, and 3-iodopropagylbutylcarbamate;
thiocyanate compounds such as 2-(4-thiocyanomethylthio)benzothiazole;
isothiazolinone derivatives such as 2-octyl-4-isothiazoline-3-one, 5-chloro-2-methyl-4-isothiazoline-3-one, 1,2-benzisothiazolone-3, and N-butyl-1,2-benzisothiazolone-3;
trihalomethylthio compounds such as N-(fluorodichloromethylthio)phtalimide, N,N-dimethyl-N'-(fluorodichloromethylthio)-N'-phenylsulfami de, and N-dichlorofluoromethylthio-N',N'-dimethyl-N-p-tolylsulfamid e;
quaternary ammonium salts such as alkyl dimethyl benzyl ammonium chloride, dodecyl dimethyl benzyl ammonium chloride, benzethonium chloride, hexadecyl trimethyl ammonium bromide, didecyl dimethyl ammonium chloride, decyl isononyl dimethyl ammonium chloride, hexadecyl pyridinium chloride (cetyl pyridinium chloride), 4,4'-(tetramethylenedicarbonylamino)bis(1-decylpyridinium bromide), and N,N'-hexamethylenebis(4-carbamoyl-1-decyl pyridinium bromide);
biguanide compounds such as polyhexamethylene biguanide hydrochloride, chlorhexidine gluconate, and chlorhexidine hydrochloride;
aldehydes such as formaldehyde, 1,5-pentanedial (glutalaldehyde), and α-bromocinnamaldehyde;
phenols such as 3-methyl-4-chlorophenol, 4-chloro-3,5-dimethylphenol, and alkyl (methyl, ethyl, propyl, butyl) p-hydroxybenzoate;
benzimidazole derivatives such as 2-(4-thiazolyl)benzimidazole and methyl 2-benzimidazolyl carbamate;
pyridine oxides such as sodium pyridine-2-thiol-1-oxide and zinc bis (2-pyridylthio-1-oxide);
carbanilides such as 3,4,4'-trichlorocarbanilide and 4,4'-dichloro-3-(trifluoro methyl)carbanilide;
diphenyl ethers such as 2,4,4'-trichloro-2'-hydroxydiphenyl ether;
carboxylic acids such as sorbic acid, propionic acid, 10-undecylenic acid, and benzoic acid; and
organometallic compounds such as 10,10'-oxybisphenoxyarsine.

Furthermore, antibacterial agents which can be used herein include: kampo medicines which show antibacterial action, such as artemisia capillaris flower, fennel, astragalus root, goldenthread rhizome, cork-tree bark, skullcap root, wormwood leaf, Chinise liquorice root, apricot kernel, Chinese cinnamon bark, houpu magnolia bark, perilla herb, Chinese peony root, cnidium root, turkey rhubarb rhizome, clove flower, peach kernel, and tree peony bark; natural antibacterial agents which are added to food, such as styrax extract, *Artemisia capillaris Thunb.* extract, Magnolia obovata extract, forsythia extract, pectin digests, protamine, polylysine, citrus seed extract, ginger extract, tea extract, raw soybean extract, digest of red breadlike food made of wheat gluten (*BENIFU*), Psoralea corylifolia L. extract, moso bamboo extract, rice hull extract, lysozyme, pepper extract, and propolis; and other natural antibacterial agents such as mustard extract.

Further more, α-polylysine and ε-polylysine may be used as the natural antibacterial agents which are added to food.

Particularly preferable antibacterial agents are cationic surfactants which absorb *Staphylococcus epidermidis,* i.e., a possible pathogen of a heat rash, and which destroys the cell membranes of the bacteria. The examples of the cationic surfactant include cetylpyridinium chloride (CPC) and the like.

Note that it is preferable that an adhesive be coated in any of various patterns including a spot-like pattern (dotted pattern), a line pattern, a spiral pattern, and the like when the adhesive is used to join the liquid-permeable sheet 17 to the diaper main body 15.

Moreover, it is preferable that the liquid-permeable sheet 17 be provided with an elastic member such as a rubber directly or indirectly. When the liquid-permeable sheet is to be provided to the skin facing side of the back waistline region, the liquid-permeable sheet 17 is less likely to come into contact with the back of the wearer since the back is recessed. Therefore, providing the elastic member securely brings the liquid-permeable sheet 17 into close contact with the back of the wearer. Alternatively, forming the liquid-permeable sheet 17 in a wave pattern or in a convexo-concave pattern securely brings the liquid-permeable sheet 17 into close contact with the back of the wearer, and brings the liquid-permeable sheet 17 into contact with the body of the wearer more frequently.

Moreover, the liquid-permeable portion which includes cellulose fibers and uses cationic antibacterial agent as an antibacterial agent may be used. With this configuration, a large amount of a cationic surfactant dissolved by the sweat remains inside the liquid-permeable portion containing the cellulose fibers, and thus exerts its antibacterial effect inside the liquid-permeable portion. This is because cationic surfactants adsorbs to cellulose fibers. Accordingly, the cationic surfactant is far less likely to reach the skin of the wearer after flowing out of the liquid-permeable portion. In this manner, it is possible to suppress an antibacterial action on *Staphylococcus epidermidis* on the skin.

Note that, in the present embodiment, the description has been given mainly on the case where the liquid-permeable portion and the antibacterial-agent-containing polymer coated portion are provided in the waistline region. However, the liquid-permeable portion and the antibacterial-agent-containing polymer coated portion may be provided in the leg-surrounding region. In this case as well, the similar effects are obtainable as in the case where these portions are provided in the waistline region. EXAMPLE CASES

Hereinbelow, the present invention will be described concretely by use of example cases.

### [Example Case 1]

Firstly, an examination was made to check how much an antibacterial-agent-containing polymer according to the present invention suppresses the growth of *Staphylococcus epidermidis.*

(1) At first, test strains shown in Table 1 below were incubated at 35°C for 20 hours by use of blood agar plates, respectively.

### [Table 1]

**TABLE.1**

| (CFU/mL) | | | | | | | | |
|---|---|---|---|---|---|---|---|---|
| Test strain | Test sample name | | Coating amount | Reaction time (duration) | | | | |
| | | | | Immediately after immersion | 2 | 4 | 8 | 24 |
| S.epidermidis NBRC12993 | Example 1 | PEG containing 1% CPC | 0.5g/m² | 1.2 × 10⁴ | 4.0 × 10¹ | <2.0 × 10¹ | <2.0 × 10¹ | <2.0 × 10¹ |
| | Example 2 | PEG containing 1% CPC | 1.0g/m² | 1.1 × 10⁴ | <2.0 × 10¹ | <2.0 × 10¹ | <2.0 × 10¹ | <2.0 × 10¹ |
| | Example 3 | PEG containing 1% CPC | 1.5g/m² | 7.8 × 10³ | <2.0 × 10¹ | <2.0 × 10¹ | <2.0 × 10¹ | <2.0 × 10¹ |
| | Comparative Example 1: growth control(1/10MHB) | | | 1.2 × 10⁴ | 1.2 × 10⁴ | 8.0 × 10³ | 7.2 × 10⁴ | 1.6 × 10⁷ |

Subsequently, each of the test strains was suspended in a sterile saline in a way that the cell density of the test strain becomes a 0.5 McFarland standard (approximately 10⁸ CFU/ml) to prepare a test bacterial suspension.

(2) The test bacterial suspension was added to 1/10 Mueller Hinton broth (MHB) in a way that the cell density of the test strain becomes approximately 10⁴ CFU/ml. Then the obtained suspension was dispensed 0.5 ml per well of a microplate having flat-bottom wells of a diameter of 24 mm.

(3) Each test sample was immersed in the liquid level of each well and left for culture at 35°C. In addition, a well in which no test sample was immersed was prepared as a growth control, which is shown as Comparative Example 1.

In Table 1, Example 1 is coated with an antibacterial-agent-containing polymer 0.5 g per 1 m², the antibacterial-agent-containing polymer obtained by mixing 1 wt% of CPC with PEG. Meanwhile, Example 2 is coated with the antibacterial-agent-containing polymer 1.0 g per 1 m², and Example 3 is coated with the antibacterial-agent-containing polymer 1.5 g per 1 m².

(4) The number of viable cells was measured by taking a culture medium in each well immediately after each test sample was immersed therein, after 2 hours of culture from the immersion, after 4 hours of culture from the immersion, after 8 hours of culture from the immersion, and after 24 hours of culture from the immersion.

As a result, as shown in Table 1, the number of viable cells decreased to almost zero in Example 1 after the elapse of 4 hours, while the number of viable cells decreased to almost zero in Examples 2 and 3 after the elapse of 2 hours. In contrast, it was confirmed that a large number of viable cells had remained in Comparative Example 1 even after the elapse of 24 hours.

### [Example Case 2]

Secondly, an examination was made to check whether or not test subjects suffer from the heat rash through the actual use of diapers.

### (Formation of Liquid-permeable Sheet)

In Example Case 2, a nonwoven fabric was used for the liquid-permeable sheet. This nonwoven fabric was formed of polyethylene terephthalate (PET) and rayon by use of a spun-lace method. The external shape thereof was a rectangle of the size 285 mm x 60 mm.

An antibacterial-agent-containing polymer was coated on a non-skin facing side of this nonwoven fabric, the antibacterial-agent-containing polymer obtained by mixing an antibacterial agent with a water-soluble polymer. Polyethylene glycol (PEG) was used as the water-soluble polymer. The PEG here is a mixture of 30 wt% of PEG with molecular mass of 600 and 70 wt% of PEG with molecular mass of 4000.

Meanwhile, cetylpyridinium chloride (CPC) was used as the antibacterial agent. The coating amount was 1.0 g/m², and the weight ratio with respect to PEG was set to PEG:CPC = 99:1. Moreover, a spray coating was employed as a coating method of the antibacterial-agent-containing polymer.

Note that the non-skin facing side of the liquid-permeable sheet was applied with a hot-melt adhesive in such a manner as to leave a dry edge by 5 mm and then was bonded to the skin facing side of the back waistline region of the diaper main body.

### (Implementation Method)

Diapers each having the liquid-permeable sheet bonded were worn by 50 test subjects around 24 months old who fit in L size diapers. Their mothers checked how the test subjects suffered from the heat rash. The test subjects were 25 males and 25 females and were extracted from infants who had already experienced the heat rash in the summer of the current year. The test was implemented during a period from August 1 to 7 where the heat rash was most likely to occur. The diapers used were mainly pants-type diapers and the number of diapers used was 25 for each test subject.

The test results are shown in Table 2 below.

### [Table 2]

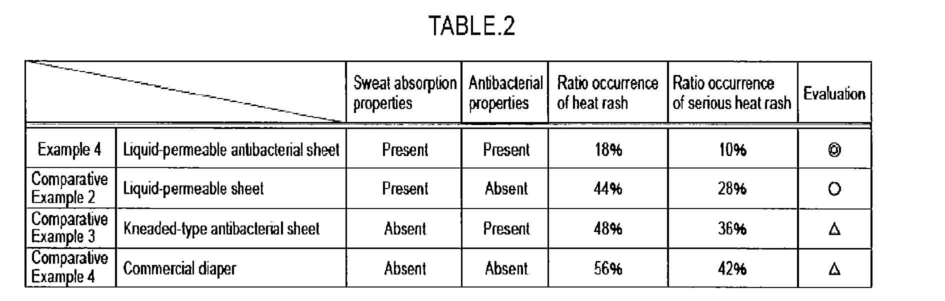

In Table 2, Example 4 shows a case of using a diaper to which a liquid-permeable sheet is pasted, the liquid-permeable sheet being coated with an antibacterial-agent-containing polymer on its non-skin facing side. Comparative Example 2 shows a case of using a diaper to which a liquid-permeable sheet is pasted, the liquid-permeable sheet not being coated with the antibacterial-agent-containing polymer. Comparative Example 3 shows a case of using a diaper to which a spun-bond nonwoven fabric is pasted, the spun-bond nonwoven fabric including polypropylene fibers containing an antibacterial agent made of silver-ion-carrying zeolite. Note that this antibacterial agent is made by Sinanen Zeomic Co., Ltd. Comparative Example 4 shows a case of using a diaper to which no liquid-permeable sheet is pasted.

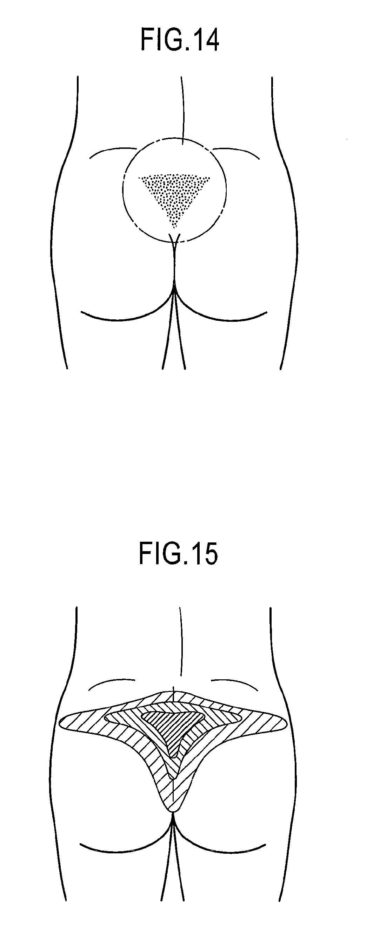

FIG. 14 is a schematic diagram showing an area from the back to the vicinity of the buttock of the test subject having participated in Example Case 2. In many cases, the heat rash occurred in an almost triangular recessed portion around the coccyx, which is circled in the figure. This is possibly because the skin on this portion has high temperature, and thus is likely to sweat easily as shown in FIG. 15, and because this portion is in a triangular recessed form and thus the sweat is likely to accumulate therein. Incidentally, in FIG. 15, the innermost isotherm surrounds an area of 36.0 to 36.5°C. FIG. 15 shows that the temperature gradually decreases toward the outermost isotherm.

As shown in Table 2, in the case of Example 4, the overall occurrence ratio of the heat rash was 18% while the occurrence ratio of a serious heat rash was as low as 10%, which were excellent. Here, the "serious heat rash" indicates a heat rash showing such a serious rash that the mother needs any kind of treatment for the test subject, including an application of medication to cure the heat rash. Note that the "occurrence ratio" is a ratio obtained by dividing the number of test subjects having suffered from the heat rash by the total number of test subjects.

Meanwhile, in Comparative Examples 2 to 4, excellent results were not obtained both in the overall occurrence ratio of the heat rash and the occurrence ratio of the serious heat rash.

Note that the entire content of Japanese Patent Application No. 2007-203376 (filed on August 3, 2007) is incorporated herein by reference.

### INDUSTRIAL APPLICABILITY

As has been described, the disposable diaper according to the present invention is useful in that an occurrence of a rash such as a heat rash can be effectively suppressed.

## Claims

1. A disposable diaper comprising a diaper main body which includes an inner surface sheet, an outer surface sheet, and a liquid absorbent core,
and which has a waistline region and a leg-surrounding region formed therein,
the inner surface sheet disposed on a skin facing side of the disposable diaper, the skin facing side facing a skin of a wearer when the disposable diaper is worn,
the outer surface sheet opposed to the inner surface sheet and disposed on a non-skin facing side which is the opposite side to the skin facing side,
the liquid absorbent core interposed between the inner surface sheet and the outer surface sheet, wherein
a liquid-permeable portion which allows moisture to permeate therethrough is provided at least partially on a skin facing side of any of the waistline region and the leg-surrounding region of the diaper main body, the skin facing side facing the skin of the wearer, and
an antibacterial-agent-containing polymer coated portion coated with an antibacterial-agent-containing polymer is provided between the outer surface sheet and a skin-facing surface of the liquid-permeable portion, the antibacterial-agent-containing polymer containing a thermoplastic water-soluble polymer and an antibacterial agent, the skin-facing surface being a surface of the liquid-permeable portion on the skin facing side.

2. The disposable diaper according to claim 1, wherein
the liquid-permeable portion is a liquid-permeable sheet being formed as a separate body from the inner surface sheet, being disposed on the skin facing side of the diaper main body, and allowing moisture to permeate therethrough, and
the antibacterial-agent-containing polymer coated portion is provided on at least one of a non-skin facing side of the liquid-permeable sheet and the skin facing side of a portion of the diaper main body, the portion facing the liquid-permeable sheet.

3. The disposable diaper according to claim 1, wherein
a folded-over portion is formed by extending and folding over at least one of end portions of the waistline region and the leg-surrounding region of the outer surface sheet, the folded-over portion being attached to the skin facing side of the diaper main body, the folded-over portion being set as the liquid-permeable portion, and
the antibacterial-agent-containing polymer coated portion is provided on at least one of a non-skin facing side of the folded-over portion and the skin facing side of a portion of the diaper main body, the portion facing the folded-over portion.

4. The disposable diaper according to claim 1, wherein
a liquid-permeable sheet which allows moisture to permeate therethrough is attached to the skin facing side of the diaper main body,
a folded-over portion is formed by extending and folding over at least one of end portions of the waistline region and the leg-surrounding region of the outer surface sheet, the folded-over portion covering at least partially a skin facing side of the liquid-permeable sheet, the liquid-permeable sheet and a portion of the folded-over portion being set as the liquid-permeable portion, the portion covering the liquid-permeable sheet, and
the antibacterial-agent-containing polymer coated portion is provided on at least one of a non-skin facing side of the portion of the folded-over portion covering the liquid-permeable sheet, the skin facing side of the liquid-permeable sheet, the non-skin facing side of the liquid-permeable sheet, and the skin facing side of a portion of the diaper main body, the portion of the diaper main body facing the liquid-permeable sheet.

5. The disposable diaper according to claim 1, wherein
at least a portion of any one of the waistline region and the leg-surrounding region in the inner surface sheet is set as the liquid-permeable portion, and
the antibacterial-agent-containing polymer coated portion is provided on at least one of a non-skin facing side of the portion of the inner surface sheet set as the liquid-permeable portion and the skin facing side of a portion of the diaper main body facing the inner surface sheet of the liquid-permeable portion.

6. The disposable diaper according to any one of claims 1 and 2, wherein
the liquid-permeable portion includes a skin facing side layer and a non-skin facing side layer, the skin facing side layer disposed on a skin facing side which faces the skin of the wearer when the disposable diaper is worn, the non-skin facing side layer disposed on a non-skin facing side of the skin facing side layer, and
hydrophilicity of the non-skin facing side layer is set higher than hydrophilicity of the skin facing side layer.

7. The disposable diaper according to any one of claims 1 and 2, wherein
the liquid-permeable portion includes a skin facing side layer, an intermediate layer, and a non-skin facing side layer, the skin facing side layer disposed on a skin facing side which faces the skin of the wearer when the disposable diaper is worn, the intermediate layer disposed on a non-skin facing side of the skin facing side layer, the non-skin facing side layer disposed on a non-skin facing side of the intermediate layer, and
hydrophilicity of the intermediate layer is set higher than hydrophilicity of the skin facing side layer and the non-skin facing side layer.

8. The disposable diaper according to any one of claims 1 to 7, wherein
the antibacterial-agent-containing polymer coated portion is formed by coating an antibacterial-agent-containing polymer in a dotted pattern, the antibacterial-agent-containing polymer containing a thermoplastic water-soluble polymer and an antibacterial agent.

9. The disposable diaper according to any one of claims 1 to 8, wherein
the liquid-permeable portion and the antibacterial-agent-containing polymer coated portion are provided in a back waistline region of the diaper main body.
